# EUROPEAN PATENT APPLICATION

(11) **EP 4 745 123 A1**
(43) Date of publication of application: **20.05.2026**
(21) Application number: 24838882.9
(22) Date of filing: 12.07.2024
(51) Int. Cl.: C07D 207/34, C07D 453/04, C07B 57/00, A61P 3/04, A61P 9/10, A61P 9/04, A61P 7/02, A61P 7/10

(54) **METHOD FOR PREPARING PYRROLE AMIDE COMPOUND AND INTERMEDIATE OF PYRROLE AMIDE COMPOUND**

(30) Priority: 12.07.2023 CN 202310858250
(71) Applicant: Sunshine Lake Pharma Co., Ltd., Dongguan, Guangdong 523000 (CN)
(72) Inventor: WANG, Jiancheng, Dongguan, Guangdong 523871 (CN); ZHANG, Yingxun, Dongguan, Guangdong 523871 (CN); YANG, Chuanwen, Dongguan, Guangdong 523871 (CN); CHEN, Hongfen, Dongguan, Guangdong 523871 (CN); ZUO, Yinglin, Dongguan, Guangdong 523871 (CN)
(74) Representative: Hoffmann Eitle
(86) International application number: PCT/CN2024/105113
(87) International publication number: WO 2025/011630

(57) **Abstract**

This invention discloses a method for preparing pyrrole amide compound and intermediate thereof. Wherein the pyrrole amide compound and/or intermediate thereof described in this invention can be used to prepare a pyrrole amide compound that serve as a mineralocorticoid receptor antagonist. Specifically, the provided preparation method has mild preparation conditions, is simple in operation, is safe and controllable, has high yield, and is suitable for industrial production.

## Description

### FIELD OF THE INVENTION

This invention relates to the field of medicinal chemistry, specifically to a method for preparing a pyrrole amide compound, and also to important intermediates thereof and their preparation methods. The pyrrole amide compound and/or intermediates thereof described in this invention can be used to prepare a pyrrole amide compound that serves as a mineralocorticoid receptor antagonist.

### BACKGROUND ART

International patent application WO2021078135A1 discloses a class of pyrrole amide compounds and uses thereof. These compounds can be used as mineralocorticoid receptor antagonists and have potential therapeutic effects on diseases such as hyperaldosteronism, hypertension, chronic heart failure, sequelae of myocardial infarction, cirrhosis, non-alcoholic steatohepatitis, chronic kidney disease, diabetic nephropathy, renal failure, fibrosis and/or stroke. Specifically, the international application discloses a compound as shown in formula (A) (i.e., compound (*S*)-*N*-(3-fluoro-4-(methylsulfonyl)phenyl)-1-(2-hydroxyethyl)-4-methyl-5-(2-(trifluoromethyl)phenyl)-1*H*-pyrrol e-3-carboxamide), and also discloses the following preparation method: using ethyl 4-methyl-5-(2-(trifluoromethyl)phenyl)-1*H*-pyrrole-3-carboxylate as a raw material, a racemic compound of formula (A) is obtained through substitution, hydrolysis, acyl chlorination, condensation, and deprotection reactions, and then the compound of formula (A) is obtained by chiral column resolution. The method uses a chiral resolution method to separate stereoisomers. This separation method has high requirements for instruments and low yield. Overall, this preparation method has high production costs, poor atom economy, significant environmental pollution, and harsh reaction conditions, making it unsuitable for industrial-scale production.

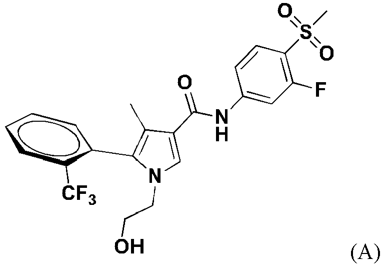

International patent application WO2022228215A1 discloses a method for preparing the compound of formula (A). In this method, lipase is used to chirally resolve the racemic raw material methyl 1-(2-hydroxyethyl)-4-methyl-5-(2-(trifluoromethyl)phenyl)-1*H*-pyrrole-3-carboxylate to obtain the intermediate (*S*)-1-(2-hydroxyethyl)-4-methyl-5-(2-(trifluoromethyl)phenyl)-1*H*-pyrrole-3-carboxylate, followed by benzyl protection, ester hydrolysis, and amination to obtain the amide intermediate (*S*)-1-(2-(benzyloxy)ethyl)-4-methyl-5-(2-(trifluoromethyl)phenyl)-1*H*-pyrrole-3-carboxamide, which is finally undergoes condensation and deprotection to obtain the compound of formula (A). This method obtains the intermediate by enzymatic resolution, avoiding the use of chiral column resolution. However, the total yield of the reaction to prepare the amide intermediate from the racemic raw material is not high, and the total yield of the compound shown in formula (A) is also not high. A method for preparing the compound shown in formula (A) with higher yield and lower cost is needed.

### SUMMARY

This invention provides intermediates which can be used to prepare the compound of formula (A) and preparation methods thereof; the intermediates include the compounds represented by the general formulae and/or structural formulae described in this invention.

This invention relates to a method for preparing a pyrrole amide compound, and also relates to important intermediates in the method and preparation methods thereof. The preparation method described in this invention is characterized by mild conditions, simple operation, safety and controllability, high yield, and is suitable for industrial production.

The pyrrole amide compound prepared by the method described in this invention can be used to prepare the compound shown in formula (A).

In one aspect, the present invention provides a compound of formula (I), 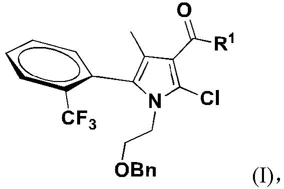 wherein, R¹ is OH, NH₂, C₁₋₄ alkoxy or benzyloxy, each of the C₁₋₄ alkoxy and benzyloxy is optionally substituted with 1, 2, 3 or 4 substituents independently selected from halogen, C₁₋₄ alkyl, C₁₋₄ haloalkyl, C₁₋₄ alkoxy and C₁₋₄ haloalkoxy.

In some embodiments, R¹ is OH, NH₂, C₂₋₄ alkoxy or benzyloxy, each of the C₂₋₄ alkoxy and benzyloxy is optionally substituted with 1, 2, 3 or 4 substituents independently selected from halogen, C₁₋₄ alkyl, C₁₋₄ haloalkyl, C₁₋₄ alkoxy and C₁₋₄ haloalkoxy.

In some embodiments, the R¹ is OH, NH₂, C₂₋₄ alkoxy (such as ethoxy, *n*-propoxy, isopropoxy, *n*-butoxy or tert-butoxy, etc.) or benzyloxy.

In other aspect, the present invention provides a compound having one of the following structures:

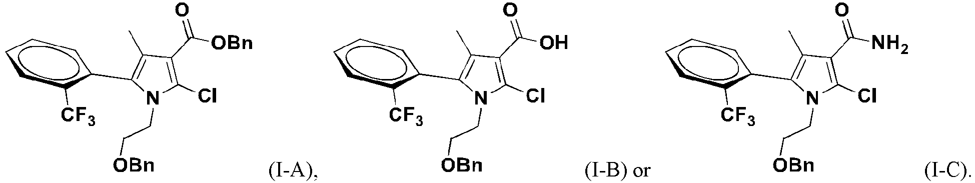

In one aspect, the present invention provides a method for preparing the compound of formula (II), 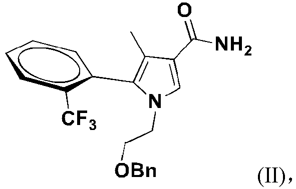 wherein, the method comprises:
Step d) The compound of formula (I-C) is dechlorinated in a solvent under suitable conditions to obtain the compound of formula (II).

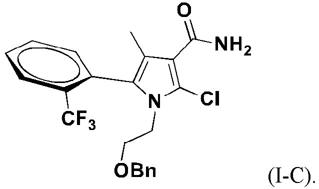

In some embodiments, the reaction solvent in step d) is methanol, ethanol, isopropanol, *tert*-butanol, or a mixed solvent composed of any of them (i.e., methanol, ethanol, isopropanol or tert-butanol) with other solvent in any combination (e.g., in any proportion). In other embodiments, the other solvent includes but is not limited to, tetrahydrofuran, DMF, ethyl acetate, methyl *tert*-butyl ether, ethylene glycol dimethyl ether, ethylene glycol monomethyl ether, toluene, water, or any combination thereof. In some embodiments, the reaction solvent in step d) is methanol, ethanol, ethanol/toluene, ethanol/water, ethanol/toluene/water, ethanol/tetrahydrofuran, or ethanol/tetrahydrofuran/water. In some embodiments, the reaction solvent in step d) is ethanol, ethanol/water or ethanol/toluene/water. Optionally, the ethanol/water in this invention refers to a mixed solvent composed of ethanol and water, and the ethanol/toluene/water refers to a mixed solvent composed of ethanol, toluene and water. Unless otherwise specified, the solvents in the mixed solvent are mixed in any proportion.

In some embodiments, the reaction solvent in step d) of the present invention is ethanol/toluene/water, wherein the volume ratio of ethanol to water is from 4:1 to 20:1, preferably from 4:1 to 18:1, more preferably from 4:1 to 17:1; even more preferably, the volume ratio of ethanol to water is from 5:1 to 16:1. In other embodiments, the reaction solvent in step d) of the present invention is ethanol/toluene/water, wherein the volume ratio of ethanol to water is 5:1, 8:1, 10:1 or 12:1.

In some embodiments, the reaction solvent in step d) of the present invention is ethanol/toluene/water, wherein the volume ratio of toluene to water is from 1:1 to 15:1, preferably from 1:1 to 12:1, more preferably from 1:1 to 11:1; even more preferably, the volume ratio of toluene to water is from 2:1 to 10:1. In other embodiments, the reaction solvent in step d) of the present invention is ethanol/toluene/water, wherein the volume ratio of toluene to water is 2:1, 4:1, 5:1, 6:1 or 10:1.

In some embodiments, the reaction solvent in step d) of the present invention is ethanol/toluene/water, wherein the volume ratio of ethanol to water is from 4:1 to 20:1, and the volume ratio of toluene to water is from 1:1 to 15:1; preferably, the volume ratio of ethanol to water is from 4:1 to 17:1, and the volume ratio of toluene to water is from 1:1 to 11:1; even more preferably, the volume ratio of ethanol to water is from 5:1 to 16:1, and the volume ratio of toluene to water is from 2:1 to 10:1. In other embodiments, the reaction solvent in step d) of the present invention is ethanol/toluene/water, wherein the volume ratio of ethanol, toluene and water is 5:10:1, 8:2:1, 5:5:1, 12:6:1, 16:4:1 or 10:10:1.

In some embodiments, the reaction in step d) of the present invention is carried out in the presence of a transition metal catalyst. Optionally, the transition metal catalyst is palladium-carbon, an other palladium-containing catalyst (such as palladium acetate), nickel or a nickel-containing catalyst (such as nickel chloride). In other embodiments, the reaction in step d) of the present invention is carried out in the presence of a transition metal catalyst, wherein the transition metal catalyst is a palladium-carbon catalyst, a palladium acetate catalyst or a nickel catalyst.

In some embodiments, the reaction in step d) of the present invention is carried out in the presence of a palladium-carbon catalyst. Optionally, the amount of the palladium-carbon catalyst is 1% to 20% of the mass of the compound of formula (I-C); in some embodiments, the amount of the palladium-carbon catalyst is 2% to 15% or 2% to 11% of the mass of the compound of formula (I-C); in some embodiments, the amount of the palladium-carbon catalyst is 3% to 10% of the mass of the compound of formula (I-C). In this invention, the reaction in step d) can achieve the target product with high yield even with a small amount of palladium-carbon catalyst, making it suitable for scale-up production.

In some embodiments, the reaction in step d) of the present invention is carried out in the presence of ammonium formate, sodium formate, sodium hydrogen phosphate (such as disodium hydrogen phosphate), acid, triethylamine or hydrogen.

In some embodiments, the reaction in step d) of the present invention is carried out in the presence of ammonium formate, sodium formate or hydrogen. In some other embodiments, the reaction in step d) of the present invention is carried out in the presence of ammonium formate or sodium formate, wherein the amount of ammonium formate or sodium formate is 1 equivalent to 5 equivalents, preferably 1 equivalent to 3 equivalents, more preferably 1 equivalent to 2 equivalents, and even more preferably 1 equivalent to 1.5 equivalents. In some other embodiments, the reaction in step d) of the present invention is carried out in the presence of sodium formate, wherein the amount of sodium formate is 1 equivalent to 2 equivalents, preferably 1 equivalent to 1.5 equivalents, and more preferably 1.1 equivalents to 1.3 equivalents. In some embodiments, the reaction in step d) of the present invention is carried out in the presence of sodium formate, wherein the amount of sodium formate is 1.2 equivalents. Optionally, the sodium formate described in this invention may be sodium formate dihydrate or anhydrous sodium formate. Unless otherwise stated, the equivalent referred to herein means the ratio of the molar amount of the reagent sodium formate to the molar amount of the reaction substrate compound of formula (I-C); for example, the expression "the amount of sodium formate is 1.2 equivalents" means that the ratio of the molar amount of sodium formate to the molar amount of the compound of formula (I-C) is 1.2 .

In some embodiments, the reaction in step d) of the present invention is carried out under heating conditions. Optionally, the heating conditions are heating to 45 °C-75 °C; in some embodiments, the heating conditions are heating to 55 °C-70 °C; in some embodiments, the heating conditions are heating to 60 °C-65 °C.

In some embodiments, the method for preparing the compound of formula (II) of the present invention further comprises a post-treatment method for the reaction in step d).

In other embodiments, the post-treatment method for the reaction in step d) of the present invention includes but is not limited to: after the reaction is completed, the reaction solution is filtered, washed, followed by concentration to remove solvents (such as ethanol and toluene), to the residue was added water, and the mixture is stirred at room temperature, filtered, the filter cake is optionally washed, collected and dried to obtain the target compound. Optionally, the filtration may be vacuum filtration or filtration through celite pad. Optionally, the first wash is performed using a reaction solvent or one thereof, such as ethanol; the second wash may optionally be performed, for example, with water. Optionally, in the post-treatment method, other solvents may be added before concentration, for example, DMAC (i.e., *N*,*N*-dimethylacetamide) may be added. Optionally, the water in "adding water" may be ambient temperature water or ice water, and the water may be added dropwise. Optionally, the drying may be vacuum drying at room temperature or vacuum drying with heating (e.g., vacuum drying at 45 °C-60 °C).

In other embodiments, the post-treatment method for the reaction in step d) of the present invention includes but is not limited to: after the reaction is completed, the reaction solution is filtered, washed, followed by concentration to remove solvents (such as toluene and ethanol), the residue is dissolved in ethanol, the pH is adjusted with hydrochloric acid, water is added dropwise, and the mixture is stirred at room temperature, filtered, the filter cake is optionally washed, collected, and dried to obtain the target compound. Optionally, the filtration may be vacuum filtration or filtration through celite pad. Optionally, the first wash is performed using a reaction solvent, such as ethanol; and the second wash may optionally be performed, for example, with water. Optionally, other solvents may be added prior to the concentration, for example, DMAC (i.e., *N*,*N*-dimethylacetamide) may be added. Optionally, the drying may be vacuum drying at room temperature or vacuum drying with heating (e.g., vacuum drying at 45 °C-60 °C).

Optionally, after the reaction in step d) is completed, other suitable post-treatment methods may be used.

The method described in this invention can effectively control the generation of reaction impurities and obtain the intermediate compound shown in formula (II) with high purity and high yield.

In some embodiments, the method for preparing the compound of formula (II) of the present invention further comprises a method for preparing the compound of formula (I-C), wherein, the method for preparing the compound of formula (I-C) comprises:
Step a) The compound of formula (I-1) reacts with a benzyl protecting group reagent to obtain the compound of formula (I-A); 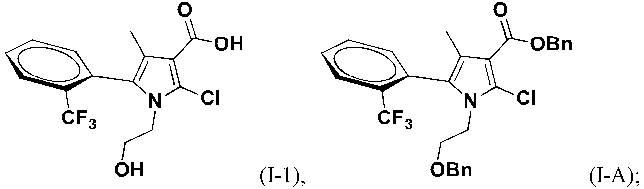
Step b) The compound of formula (I-A) is debenzylated under suitable conditions to obtain the compound of formula (I-B);

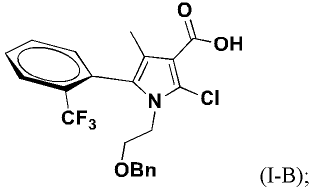

Step c) The compound of formula (I-B) is aminated under suitable conditions to obtain the compound of formula (I-C).

In some embodiments, the benzyl protecting group reagent in step a) is a halobenzyl group, optionally benzyl bromide.

In some embodiments, step a) is carried out in the presence of sodium *tert*-amylate.

In some embodiments, the reaction solvent in step a) is *N*,*N*-dimethylacetamide.

In some embodiments, the reaction temperature in step a) is 25 °C to 60 °C. In other embodiments, the reaction temperature in step a) is 35 °C to 55 °C. In other embodiments, the reaction temperature in step a) is 35 °C to 45 °C. In other embodiments, the reaction temperature in step a) is 45 °C to 55 °C.

In some embodiments, the reaction in step b) is carried out in the presence of a base, and the base is sodium carbonate, potassium carbonate, cesium carbonate, potassium phosphate, lithium hydroxide, potassium hydroxide or sodium hydroxide.

In some embodiments, the reaction in step b) is carried out in a solvent, and the solvent is acetone, acetonitrile, *N*,*N*-dimethylformamide, *N*,*N*-dimethylacetamide, DMSO, methanol, ethanol, THF, methyl *tert*-butyl ether, water or any combination thereof.

In some embodiments, the reaction in step b) is carried out under heating conditions, and the heating conditions mean heating to 60 °C to 80 °C; in other embodiments, the heating conditions mean heating to 65 °C to 75 °C.

In some embodiments, in step c), the compound of formula (I-B) reacts with thionyl chloride, oxalyl chloride or *N*,*N*'-carbonyldiimidazole (i.e., 1-(1*H*-imidazolium-1-carbonyl)-1*H*-imidazolium) and then reacts with an amination reagent to obtain the compound of formula (I-C); optionally, the amination reagent is ammonia or an ammonium salt reagent. In other embodiments, the amination agent is ammonia, ammonium bromide or NH₄SCN.

In some embodiments, the reaction in step b) is carried out in a solvent, and the solvent is *N*,*N*-dimethylformamide or *N,N*-dimethylacetamide. In other embodiments, the reaction in step d) of the present invention is carried out at room temperature.

In other aspect, the present invention provides the use of the compound of formula (I) in the preparation of a pyrrole amide compound used as a mineralocorticoid receptor antagonist. 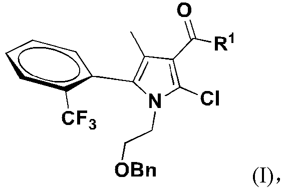 wherein, R¹ is OH, NH₂, C₂₋₄ alkoxy or benzyloxy, and each of the C₂₋₄ alkoxy and benzyloxy is optionally substituted with 1, 2, 3 or 4 substituents independently selected from halogen, C₁₋₄ alkyl, C₁₋₄ haloalkyl, C₁₋₄ alkoxy and C₁₋₄ haloalkoxy.

In some embodiments, the present invention provides the use of a compound having one of the following structures in the preparation of a pyrrole amide compound used as a mineralocorticoid receptor antagonist.

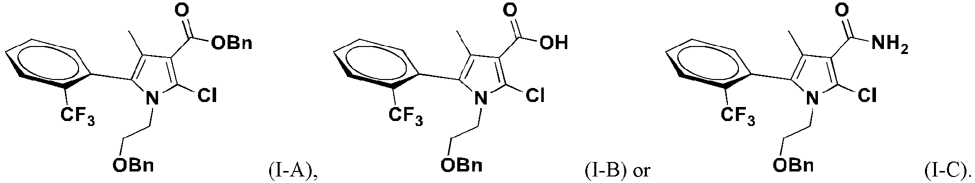

In some embodiments, the pyrrole amide compound used as a mineralocorticoid receptor antagonist according to the present invention is (*S*)-*N*-(3-fluoro-4-(methylsulfonyl)phenyl)-1-(2-hydroxyethyl)-4-methyl-5-(2-(trifluoromethyl)phenyl)-1*H*-pyrrol e-3-carboxamide (i.e., the compound of formula (A)).

In one aspect, the present invention provides a method for preparing the compound of formula (A) (i.e., (*S*)-N-(3-fluoro-4-(methylsulfonyl)phenyl)-1-(2-hydroxyethyl)-4-methyl-5-(2-(trifluoromethyl)phenyl)-1*H*-pyrrol e-3-carboxamide), 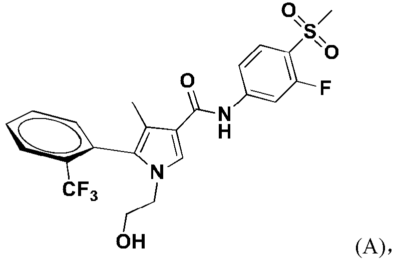 wherein, the method comprises:
The compound of formula (II) reacts with the compound of formula (III) to obtain the compound of formula (IV), and the compound of formula (IV) undergoes a deprotection reaction to remove the benzyl group to obtain the target compound; 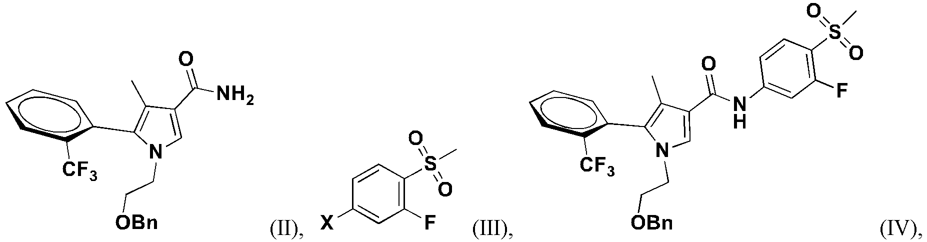 wherein, X is Br or I.

Optionally, the method for preparing the compound of formula (A) of the present invention further comprises a method for preparing the compound of formula (II) as described in the present invention.

In some embodiments, the deprotection reaction of the present invention is carried out in a hydrogen atmosphere in the presence of palladium-carbon.

In some embodiments, the deprotection reaction of the present invention may be carried out under acidic conditions or in the presence of acidic substances.

In some embodiments, the deprotection reaction of the present invention is carried out in the presence of a Lewis acid. In still other embodiments, the Lewis acid is zinc chloride (ZnCl₂), titanium tetrachloride or aluminum chloride, etc.

In yet other embodiments, the acidic conditions refer to the presence of an acid suitable for the deprotection reaction, including but not limited to hydrochloric acid, acetic acid, trifluoroacetic acid, etc.; preferably, the acid is hydrochloric acid, sulfuric acid, trifluoroacetic acid or a solution thereof.

In still other embodiments, in the deprotection reaction of the present invention, the amount of the acid is from 0.10 mL/g to 0.40 mL/g relative to the mass of the compound of formula (IV). In still other embodiments, in the deprotection reaction of the present invention, the amount of the acid is from 0.10 mL/g to 0.20 mL/g relative to the mass of the compound of formula (IV).

In some other embodiments, the acid described in this invention is hydrochloric acid, and the amount of hydrochloric acid is from 0.10 mL/g to 0.40 mL/g. In still other embodiments, the hydrochloric acid refers to an aqueous solution of hydrogen chloride. Specifically, the concentration of the hydrochloric acid is greater than 1 mol/L; preferably, the concentration of the hydrochloric acid is from 1 mol/L to 12 mol/L; more preferably, the concentration of the hydrochloric acid is from 3 mol/L to 12 mol/L.

In some embodiments, the reaction temperature of the deprotection reaction of the present invention is 50 °C-75 °C; in some embodiments, the reaction temperature of the deprotection reaction of the present invention is 55 °C-70 °C.

In some embodiments, the deprotection reaction of the present invention may be carried out at atmospheric pressure (0.1 MPa) or at a pressures higher than atmospheric pressure. In other embodiments, the pressure of the deprotection reaction of the present invention is from 0.1 MPa to 3.8 MPa. In still other embodiments, the pressure of the deprotection reaction of the present invention is from 0.8 MPa to 3.8 MPa.

In some embodiments, the deprotection reaction of the present invention is carried out in an alcohol solvent, wherein the alcohol solvent is methanol, ethanol, isopropanol, *tert*-butanol or *tert-amyl* alcohol. Optionally, the amount of the alcohol solvent may be 10 mL/g, based on the mass of the compound of formula (IV).

In some embodiments, the crude product obtained after the deprotection reaction of the present invention is purified by recrystallization to obtain the pure compound of formula (A). In other embodiments, the recrystallization of the present invention is carried out in a mixed solvent of ethanol and water. In still other embodiments, the volume ratio of ethanol to water may be any suitable ratio, including but not limited to 1:2-1:15. In still other embodiments, the recrystallization of the present invention may be carried out in a mixed solvent of isopropyl acetate and toluene. In still other embodiments, the volume ratio of isopropyl acetate to toluene may be any suitable ratio, including but not limited to 1:2-1:15; specifically, the volume ratio of isopropyl acetate to toluene may be 1:5.

The purification of the compound of formula (A) in the present invention may be achieved by multiple recrystallizations, and the conditions for each recrystallization may be the same or different. When necessary, the compound of formula (A) of the present invention may be further purified by other conventional purification methods in the art to remove impurities, for example, by using palladium-removing silica gel and/or activated carbon to remove residual palladium. The recrystallization may be performed by firstly dissolving the crude product in a good solvent, stirring to dissolve it, and then adding a poor solvent, or by adding the solution of the crude product to a poor solvent, and stirring to precipitate a solid. The dissolution process may be carried out at room temperature or under heating conditions. Preferably, after dissolving the crude product in a good solvent, impurities may be removed by means such as palladium-removing silica gel and/or activated carbon, and then the solution of the crude product may be added to a poor solvent to precipitate a solid. Preferably, in the above recrystallization, the good solvent may be ethanol or isopropyl acetate, and the poor solvent may be water or toluene. In some embodiments, the good solvent is ethanol and the poor solvent is water; preferably, the total volume ratio of ethanol to water is from about 1:1.5 to about 1:15, specifically from about 1:1.8 to about 1:15. In still other embodiments, the good solvent is isopropyl acetate and the poor solvent is toluene; preferably, the total volume ratio of isopropyl acetate to toluene is from about 1:2 to about 1:15, specifically from about 1:4 to about 1:5.

In some embodiments, the reaction of the compound of formula (II) with the compound of formula (III) is carried out in the presence of a base. Preferably, the base is potassium carbonate, cesium carbonate, sodium carbonate, potassium *tert*-butoxide or potassium phosphate. In other embodiments, the amount of the base is a multiple of the molar amount of the compound of formula (II), including but not limited to 1.2 to 6.0 times; in still other embodiments, the amount of the base is 1.5 to 3.0 times, preferably 1.5 to 2.0 times, the molar amount of the compound of formula (II).

In some embodiments, the reaction of the compound of formula (II) with the compound of formula (III) is carried out in the presence of a catalyst, and the catalyst is a transition metal catalyst. In other embodiments, the catalyst is a palladium catalyst. Preferably, the palladium catalyst is Pd₂(dba)₃, Pd(dppf)Cl₂.CH₂Cl₂ or Pd(OAc)₂. In other embodiments, the amount of the catalyst is 0.5% to 5%, preferably 1% to 3%, more preferably 1.5% to 3%, of the molar amount of the compound of formula (II).

In some embodiments, the reaction of the compound of formula (II) with the compound of formula (III) is carried out in the presence of a ligand. In other embodiments, the ligand is DMEDA, Xantphos, X-phos or S-phos. In other embodiments, the amount of the ligand is 0.5% to 5%, preferably 1% to 3%, more preferably 1.5% to 3%, of the molar amount of the compound of formula (II).

In some embodiments, the reaction of the compound of formula (II) with the compound of formula (III) is carried out in the presence of a catalyst and a ligand. In other embodiments, the molar ratio of the catalyst to the ligand is 1:1. In other embodiments, the catalyst is Pd(OAc)₂, and the ligand is Xantphos, S-phos, or X-phos; or, the catalyst is Pd₂(dba)₃, and the ligand is Xantphos, S-phos or X-phos; or, the catalyst is Pd(dppf)Cl₂.CH₂Cl₂, and the ligand is Xantphos, S-phos or X-phos.

In some embodiments, the reaction of the compound of formula (II) with the compound of formula (III) is carried out in a solvent, wherein the solvent is toluene, dioxane, *tert*-butanol (t-BuOH), *tert-amyl* alcohol (*t*-AmOH), dimethyl ether (DME), cyclopentyl methyl ether (CPME), *N*,*N*-dimethylacetamide (DMAC), *N*,*N*-dimethylformamide (DMF), water or any combination thereof.

In some embodiments, the reaction temperature of the compound of formula (II) with the compound of formula (III) is 55 °C-110 °C, more preferably 55 °C-100 °C, and more preferably 55 °C-83 °C.

In some embodiments, the crude product of the compound of formula (IV) prepared according to the method described in this invention may be purified by purification methods. The purification methods include, but are not limited to, recrystallization, removal of impurities by using palladium-free silica gel and/or activated carbon; if necessary, multiple recrystallizations may be performed. The recrystallization may be performed by firstly dissolving the crude product in a good solvent, stirring to dissolve it, and then adding a poor solvent, or by adding the solution of the crude product to a poor solvent, and stirring to precipitate a solid. The dissolution process may be carried out at room temperature or under heating conditions. Preferably, after dissolving the crude product in a good solvent, impurities may be removed by means such as palladium-removing silica gel and/or activated carbon, and then the solution of the crude product may be added to a poor solvent to precipitate a solid. In some embodiments, the good solvent is ethanol and the poor solvent is water; preferably, the total volume ratio of ethanol to water is from about 1:1.5 to about 1:15, specifically from about 1:1.8 to about 1:15.

In one aspect, the method for preparing the compound of formula (II) or the method for preparing the compound of formula (I-C) according to the present invention further comprises a method for preparing the compound of formula (I-1). 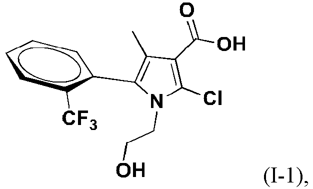 wherein, the preparation method of the compound of formula (I-1) comprises:
1a) The compound of formula (I-0) reacts with an optically active amine to obtain the corrsponding salt of the optically active amine of the compound of formula (I-1), and
1b) The salt obtained in step 1a) reacts to give the compound of formula (I-1);

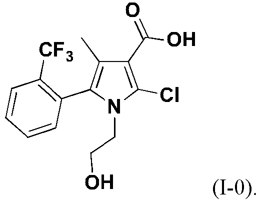

In some embodiments, the optically active amine of the present invention is an optically active amine having a quinine skeleton.

In some embodiments, the optically active amine havin a quinine skeleton of the present invention is quinine, hydroquinine, quinidine, cinchonine or cinchonidine.

In some embodiments, the salt of the optically active amine of the compound described in Formula (I-1) of the present invention is the salt formed by the compound shown in Formula (I-1) and the optically active amine. In other embodiments, the salt of an optically active amine of the compound of Formula (I-1) of the present invention is a quinine salt of the compound of Formula (I-1), a hydroquinine salt of the compound of Formula (I-1), a quinidine salt of the compound of Formula (I-1), a cinchonine salt of the compound of Formula (I-1), or a cinchonidine salt of the compound of Formula (I-1).

In some embodiments, the reaction in step 1b) of the present invention is a hydrolysis reaction.

In some embodiments, the reaction in step 1b) of the present invention is carried out under acidic conditions.

In some embodiments, the acidic conditions of the present invention are the conditions for the presence of hydrochloric acid, sulfuric acid, hydrobromic acid or citric acid.

In some embodiments, the reaction solvent in step 1b) of the present invention is *N*,*N*-dimethylformamide (DMF), *N*,*N*-dimethylacetamide (DMAC), acetonitrile, tetrahydrofuran (THF), ethanol, acetone, isopropyl acetate, ethyl acetate, methyl *tert*-butyl ether, water, dimethoxyethane or any combination thereof.

In some embodiments, the reaction solvent in step 1a) of the present invention is *N*,*N*-dimethylformamide (DMF), *N*,*N*-dimethylacetamide (DMAC), acetonitrile, tetrahydrofuran (THF), ethanol, acetone, isopropyl acetate, ethyl acetate, methyl *tert*-butyl ether, water, dimethoxyethane or any combination thereof.

In some embodiments, the reaction temperature in step 1a) of the present invention is room temperature to 100 °C; in some embodiments, the reaction temperature in step 1a) of the present invention is 50 °C-80 °C; in some embodiments, the reaction temperature in step 1a) of the present invention is 55 °C-65 °C.

In some embodiments, the preparation method of the compound of Formula (I-1) of the present invention further comprises: spinning the mixed solution obtained after removing the salt of an optically active amine of the compound of Formula (I-1) in step 1a), adding a suitable solvent, isomerizing by heating to obtain a racemic compound of Formula (I-0), then reacting with an optically active amine to obtain a salt of the optically active amine of the compound of Formula (I-1). In general, the optically active amine described here is the same as that used in step 1a). Optionally, the suitable solvent includes but is not limited to, DMF, etc.

In other embodiments, optionally, the isomerization refers to the racemization of the atropisomer in a solution by a mean such as heating to obtain racemic compound (i.e., the compound of Formula (I-0)). Optionally, a salt of an optically active amine of the compound of Formula (I-1) may be prepared by the racemic compound through the method of step 1a) of the present invention. Optionally, the compound of Formula (I-1) may be prepared by the salt of an optically active amine of the compound of Formula (I-1) through the method of step 1b) of the present invention.

The preparation method of the compound of Formula (I-1) shown in the present invention is simple to operate, and has high yield and high ee/de value of the obtained product; and the by-product of R configuration obtained by this method may be recycled through the racemization decribed in the present invention, thereby further improving the yield of the compound of Formula (I-1) (up to more than 60%).

In other aspect, the present invention provides a salt formed by the compound shown in Formula (I-1) and an optically active amine,

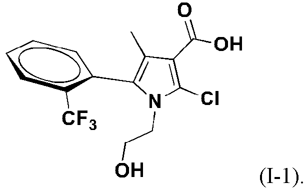

In some embodiments, the optically active amine of the present invention is quinine, hydroquinine, quinidine, cinchonine or cinchonidine. Optionally, the optically active amines have the following structures, respectively.

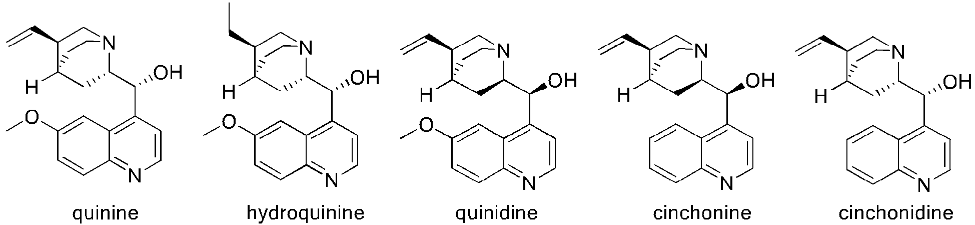

Preferably, the salt formed by the compound of Formula (I-1) of the present invention and an optically active amine is a quinine salt of the compound of Formula (I-1), a hydroquinine salt of the compound of Formula (I-1), a quinidine salt of the compound of Formula (I-1), a cinchonine salt of the compound of Formula (I-1), or a cinchonidine salt of the compound of Formula (I-1).

The compounds of Formulas (I-0), (I-1), (I-A), (I-B), (I-C), (II) and/or (IV) of the present invention are used to prepare the compound of Formula (A) of the present invention. Preferably, the method for preparing the compound shown in Formula (A) using the aforementioned compounds is as described in the present invention.

### DEFINITIONS AND GENERAL TERMINOLOGY

In the present invention, "room temperature" refers to the temperature from about 10°C to about 40°C. In some embodiments, "room temperature" refers to a temperature from about 20 °C to about 30 °C; in other embodiments, "room temperature" refers to 20°C, 22.5°C, 25°C, 27.5°C and the like.

In the context of the present invention, all values disclosed herein are approximate. The value of each number may vary by 1%, 2%, 5%, 7%, 8% or 10%. When a number with an N value is made public, any number within N +/- 1%, N +/- 2%, N +/- 3%, N +/- 5%, N +/- 7%, N +/- 8%, or N +/- 10% will be opened clearly, wherein "+/-" means plus or minus. Whenever a lower bound, DL, and an upper limit, DU, of a range of values are disclosed, any values that fall within that range are explicitly disclosed.

After the reaction proceeds to a certain extent in the present invention, such as the raw material is consumed about more than 70%, more than 80%, more than 90%, more than 95%, or completely by monitoring, the reaction mixture is worked up, such as cooled, collected, drawn, filtered, separated, purified or a combination thereof. The reaction can be monitored by conventional method such as thin-layer chromatography (TLC), high performance liquid chromatography (HPLC), gas chromatography (GC), and the like. The reaction mixture can be worked up by conventional method, for example, the crude product can be collected by concentrating the reaction mixture through vacuum evaporation or conventional distillation and is used directly in the next operation; or the crude product can be obtained by filtration of the reaction mixture and is used directly in the next operation; or the crude product can be get by pouring the supernatant liquid of the reaction mixture after standing a while and which is used directly in the next operation; or crude products can be obtained by selecting appropriate organic solvents or their combinations for extraction, distillation, crystallization, column chromatography, rinsing, pulping and other purification steps.

The term "approximately" or "about" in the present invention is used to modify a numerical value with a difference of 10% between the top and the bottom. The term "approximately" or "about" in the present invention is used to modify a numerical value with a difference of 5% between the top and the bottom. The term "approximately" or "about" in the present invention is used to modify a numerical value with a difference of 3% or 2% or 1% between the top and the bottom. It is understandable that the numerical margin of error of an "approximately" or "about" modification is an actual or reasonable margin of error depending on the value it modifies.

The term "optional" or "optionally" refers to that a subsequently described event or circumstance may but need not occur, i.e., the description includes instances where the event or circumstance occurs and instances in which it does not.

In each step of the reaction process described in the present invention, the reaction raw material or other reagent can be added to the reaction system by dropping. The dropping process and each step reaction are carried out under certain temperature conditions, and any temperature suitable for being used in each dropping process or each reaction process is included in the present invention. Additionally, many similar modifications in the art, equivalent replacements, or temperature and temperature scope which are equivalent to those described in the invention, all are deemed to be included in the present invention. The invention gives the optimal temperature or temperature range of each dropping process, and the optimal reaction temperature or reaction temperature range of each reaction.

The expressions "solvent 1", "solvent 2", "solvent a", "solvent b", "baes a", "base b" and the like described in the present invention use Arabic numerals 1, 2, 3...... after "solvent" or "base" or the letters a, b, c...... , just to better distinguish the solvents or bases used in the individual steps, the Arabic numerals or letters used have no special meaning. For example, solvent 1, includes any solvent suitable for the reaction of a compound of Formula (II) prepared by the reaction of a compound having Formula (III) with a compound shown in Formula (IV), including, but not limited to, toluene, dioxane, dimethyl sulfoxide, tert-butanol, tert-amyl alcohol, dimethyl ether (DME), cyclopentyl methyl ether (CPME), *N*,*N*-dimethylacetamide, water or any combination thereof.

The solvent used for the reaction of the invention is not particularly restricted, any solvent is contained in the invention so long as it can dissolve the raw materials to a certain extent and doesn't inhibit the reaction. Additionally, many similar modifications in the art, equivalent replacements, or solvent, solvent composition and the solvent composition with different proportions which are equivalent to those described in the invention, all are deemed to be included in the present invention. The present invention gives the preferred solvent for each reaction step.

The product of each reaction step of the present invention can be purified by recrystallization under suitable conditions. The solvent used for the recrystallization is not particularly restricted, any solvent is contained in the invention so long as it can dissolve the crude product and the recrystallized product can precipitate out under certain conditions. Additionally, many similar modifications in the art, equivalent replacements, or solvent, solvent composition and the solvent composition with different proportions which are equivalent to those described in the invention, all are deemed to be included in the present invention. Wherein the solvent could be alcohol, ethers, alkanes, halohydrocarbons, esters, ketones, aromatic hydrocarbons, acetonitrile, acetic acid, water, DMF, or a combination thereof. Such as water, acetic acid, methanol, ethanol, n-propanol, i-propanol, n-butanol, i-butanol, tert-butanol, petroleum ether, n-pentane, n-hexane, n-heptane, cyclohexane, DMF, N, N-dimethylacetamide, tetrahydrofuran, ethyl ether, isopropyl ether, dioxane, methyl tertiary butyl ether, dimethoxylethane, diethylene glycol dimethyl ether, triethylene glycol dimethyl ether, dichloromethane, 1,2-dichloroethane, chloroform, tetrachloromethane, ethyl acetate, isopropyl acetate, acetone, butanone, benzene, toluene, xylene or a combination thereof.

The amount of water in the solvent described in the present invention is not particularly restricted, that is, the content of water in the solvent does not affect the occurrence of the reaction described in the present invention. So long as the solvent containing a certain amount of water can be used in the reaction disclosed herein, which is deemed to be included in the present invention. The amount of water in the solvent is approximately less than 0.05%, less than 0.1%, less than 0.2%, less than 0.5%, less than 5%, less than 10%, less than 25%, less than 30%, or 0%. In some embodiments, the amount of water in the solvent is within a certain range, which is more conducive to the progress of the reaction; for example, in the step where ethanol is used as the reaction solvent, absolute ethanol is used to facilitate the reaction. In some embodiments, the amount of water in the solvent is outside a certain range, which may affect the progress of the reaction (e.g., affect the yield of the reaction), but does not affect the occurrence of the reaction.

### GENERAL SYNTHETIC PROCEDURES

In the present invention, if the chemical name of the compound doesn't match the corresponding structure, the compound is characterized by the corresponding structure.

In the examples described below, unless otherwise indicated all temperatures are set forth in degrees ( °C) Celsius. Unless otherwise indicated, all kinds of materials and reagents are purchased from commodity suppliers and are not further purified when used, and some materials can be prepared according to well-known methods in the art.

1H NMR spectra were recorded by a Bruker Avance 400 MHz spectrometer or Bruker Avance III HD 600 spectrometer, using CDCl₃, *d*₆-DMSO, CD₃OD or *d*₆-acetone (reported in ppm) as solvent, and using TMS (0 ppm) or chloroform (7.25 ppm) as the reference standard. When peak multiplicities are reported, the following abbreviations are used: s (singlet), d (doublet), t (triplet), m (multiplet), br (broadened), dd (doublet of doublets), dt (doublet of triplets), td (triplet of doublets), ddd (doublet of doublet of doublets), ddt (doublet of doublet of triplets), dddd (doublet of doublet of doublet of doublets). Coupling constants, when given, were reported in Hertz (Hz).

Low-resolution mass spectral (MS) data were also determined on an Agilent 6320 series LC-MS spectrometer equipped with G1312A binary pumps and a G1316A TCC (Temperature Control of Column, maintained at 30 °C), a G1329A autosampler and a G1315B DAD detector were used in the analysis. An ESI source was used on the LC-MS spectrometer.

Low-resolution mass spectral (MS) data were also determined on an Agilent 6120 series LC-MS spectrometer equipped with G1311A binary pumps, a G1316A TCC (Temperature Control of Column, maintained at 30 °C), a G1329A autosampler and a G1315D DAD detector were used in the analysis. An ESI source was used on the LC-MS spectrometer.

Both LC-MS spectrometers were equipped with an Agilent Zorbax SB-C18, 2.1 x 30 mm, 5 µm column. Injection volume was decided by the sample concentration. The flow rate was 0.6 mL/min. The HPLC peaks were recorded by UV-Vis wavelength at 210 nm and 254 nm. The mobile phase was 0.1% formic acid in acetonitrile (phase A) and 0.1% formic acid in ultrapure water (phase B). The gradient elution conditions were showed in Table 1:

**Table 1: The gradient condition of the mobile phase in Low-resolution mass spectrum analysis**

| Time (min) | A (CH₃CN, 0.1% HCOOH) | B (H₂O, 0.1% HCOOH) |
|---|---|---|
| 0 - 3 | 5 - 100 | 95 - 0 |
| 3 - 6 | 100 | 0 |
| 6 - 6.1 | 100 - 5 | 0 - 95 |
| 6.1 - 8 | 5 | 95 |

The data of the compound purity, reaction product content, or intermediate control product content are obtained by high performance liquid chromatography (HPLC). The high performance liquid chromatography (HPLC) instrument can be an Agilent HPLC system, and the chromatographic column can be Xbridge Phenyl (4.6×150mm, 3.5 µm), ZORBAX Extend-C18 (4.6×150mm, 5 µm), or Waters Xbridge phenyl (4.6×150mm, 3.5 µm); gradient elution is performed using an aqueous phosphoric acid solution (optionally containing potassium dihydrogen phosphate) and acetonitrile as the mobile phase.

The stereoisomers of the present invention are detected using high-performance liquid chromatography. The high-performance liquid chromatography (HPLC) instrument can be an Agilent HPLC system, and the chromatographic column can be selected from OJ-RH (4.6×250mm, 5 µm), Daicel CHIRALPAK IC (4.6×250mm, 5 µm), or Philomans CHIRAL NY (4.6×250mm, 5 µm); gradient elution is performed using trifluoroacetic acid-acetonitrile solution and *n*-hexane as the mobile phase.

### EXAMPLES

The embodiments of the present invention disclose methods for preparing the pyrrole amide compound as shown in Formula (A) and intermediates thereof. Skilled in the art can learn from this invention to properly improve the process parameters to implement the preparation method. Of particular note is that all similar substitutions and modifications to the skilled person is obvious, and they are deemed to be included in the present in invention. Related person can clearly realize and apply the techniques disclosed herein by making some changes, appropriate alterations or combinations to the methods without departing from spirit, principles and scope of the present disclosure.

In order to enable those skilled in the art to further understand the invention, it is detailed below through examples.

### Example

### Example 1 Preparation of 2-chloro-1-(2-hydroxyethyl)-4-methyl-5-(2-(trifluoromethyl)phenyl)-1H-pyrrole-3-carboxylic acid (intermediate 1)

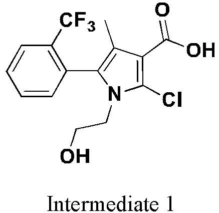

Ethyl 2-chloro-1-(2-hydroxyethyl)-4-methyl-5-(2-(trifluoromethyl)phenyl)-1*H*-pyrrole-3-carboxylate (29.1g, 77.44 mmol) prepared with reference to a well-known method in the art was added to a mixed solvent of methanol (90 mL) and water (30 mL), and then potassium hydroxide (15.34 g, 232.32 mmol, purity 85%) was added and the mixture was heated to reflux for 3 hours. The solvent was evaporated under reduced pressure, water (90 mL) was added to the residue, then the mixture was washed with methyl tert-butyl ether (90 mL), the aqueous phase was adjusted to pH=4 with 2 mol/L hydrochloric acid solution , and then extracted with ethyl acetate (150 mL × 2), the organic phases were combined, and washed with water (100 mL) and saturated brine (100 mL) successively, then dried over anhydrous sodium sulfate and filtered with suction, and the filtrate was concentrated under reduced pressure to obtain a light yellow solid of 26.3 g with a yield of 97.67%.

### Example 2 Preparation of (S)-2-chloro-1-(2-hydroxyethyl)-4-methyl-5-(2-(trifluoromethyl)phenyl)-1H-pyrrole-3-carboxylic acid (intermediate 2)

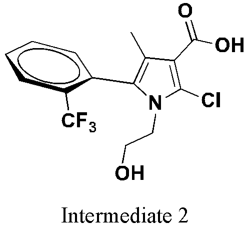

### Method one:

Quinine (7.28 g, 22.43 mmol) was weighed into a reaction flask, *N, N*-dimethylacetamide (6.5 mL), ethyl acetate (65 mL) and water (3.9 mL) were added to the flask and the mixture was heated to 60°C. 2-Chloro-1-(2-hydroxyethyl)-4-methyl-5-(2-(trifluoromethyl)phenyl)-1*H*-pyrrole-3-carboxylic acid (intermediate 1) (13.00 g, 37.39 mmol) was added to a solution of *N, N*-dimethylacetamide (6.5 mL) and ethyl acetate (48 mL), and the mixture was heated and dissolved, and then added dropwise to the aforementioned quinine solution. The heating was turned off, the system was cooled to room temperature, filtered with suction, and the filter cake was rinsed with ethyl acetate (13mL×2) and vacuum-dried. The filter cake was added to the mixed solvent of ethyl acetate (80 mL) and water (60 mL), to which was added 2 mol/L hydrochloric acid solution (18.7 mL) under stirring, the aqueous phase was separated after 10 minutes, the organic phase was washed with water (50 mL) and brine (40 mL) in turn and dried over anhydrous sodium sulfate and filtered with suction, and the filtrate was concentrated under reduced pressure to obtain a light yellow solid of 6.35 g, with an ee value of 96.04%, a purity of 99.65% and a yield of 48.84%.

### Method two:

Quinine (83.97 g, 172.55 mmol) was added into *N*,*N*-dimethylacetamide (75 mL), ethyl acetate (1050 mL) and water (45 mL), after which the mixture was heated to 65 °C. 2-Chloro-1-(2-hydroxyethyl)-4-methyl-5-(2-(trifluoromethyl)phenyl)-1*H*-pyrrole-3-carboxylic acid (intermediate 1) (150.0 g, 287.59 mmol) was added to a solution of *N*,*N-*dimethylacetamide (75 mL) and ethyl acetate (255 mL), and the mixture was heated and dissolved, and then added dropwise to the aforementioned quinine solution. After the addition was complete, the mixture was kept at 65 °C with stirring for 1 hour and then cooled to room temperature. The mixture was filtered under suction, and the filter cake was washed with ethyl acetate (50 mL × 2), collected and dried under vacuum at 45 °C for 11 hours to obtain a white solid (131.96 g). 4 M hydrochloric acid solution was added to the obtained white solid until the pH ≤3, and the mixture was stirred at room temperature for 5 hours. The mixture was filtered under suction, and the filter cake was washed with water (500 mL), collected and vacuum dried at 60 °C for 17 hours to obtain the target product as a white solid (64.83 g, 43.22%).

Identification of intermediate 2:
MS (ESI, pos. ion) m/z: 314.2 (M+1);
¹H NMR (400 MHz, CDCl3) δ 7.82 (d, *J=* 7.6 Hz, 1H), 7.64 (dt, *J=* 21.3, 7.4 Hz, 2H), 7.43 (d, *J*= 7.3 Hz, 1H), 4.05 (dt, *J =* 10.5, 6.0 Hz, 1H), 3.84 - 3.60 (m, 3H), 2.01 (s, 3H).

### Example 3 Preparation of (S)-methyl 1-(2-(benzyloxy)ethyl)-2-chloro-4-methyl-5-(2-(trifluoromethyl)phenyl)-1H-pyrrole-3-carboxylic acid benzyl ester (intermediate 3)

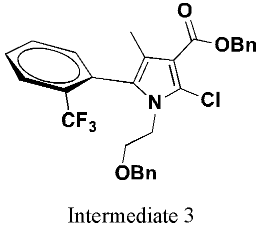

### Method one:

The intermediate 2 (100 g, 287.59 mmol) and benzyl bromide (147.56 g, 862.77 mmol) were dissolved in N,N-dimethylacetamide (2 L) under nitrogen protection, and sodium tert-amylate (95.02 g, 862.77 mmol) was added. The mixture was then heated to 40 °C and reacted overnight. The reaction solution was poured into water (4000 mL), and then extracted with MTBE (800 mL × 3). The combined organic phases were washed with saturated brine (600 mL × 2), and the solvent was removed by concentration under reduced pressure to give a pale yellow, transparent oil (151 g, 99.45%).

### Method two

The intermediate 2 (8.25 g, 23.73 mmol), benzyl bromide (12.18 g, 71.19 mmol), *N*,*N*-dimethylacetamide (83 mL) and sodium tert-amylate (7.84 g, 71.19 mmol) were added to the reaction flask. Afterward, the mixture was replaced with nitrogen three times, and then heated to 50 ± 5 °C for 4 hours under nitrogen protection. The reaction mixture was diluted with water (250 mL), extracted with methyl *tert*-butyl ether (75 mL × 2), and the organic phase was washed with 20% saline solution (200 mL × 2) and concentrated under reduced pressure to obtain the target product as a pale yellow transparent oil (12.52 g, 99.95%).

A scale-up experiment was carried out according to the method described in this example. With a feed amount of 2.2 kg of intermediate 2, the target product could still be obtained with high yield and high purity, which was 3.34 kg of a pale yellow oil with a yield of 100%.

### Example 4 Preparation of (S)-methyl 1-(2-(benzyloxy)ethyl)-2-chloro-4-methyl-5-(2-(trifluoromethyl)phenyl)-1H-pyrrole-3-carboxylic acid (intermediate 4)

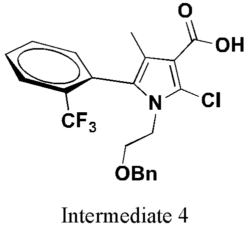

Ethanol (240 mL), potassium hydroxide (35.01 g, 530.36 mmol, 85% purity) and water (60 mL) were added to intermediate 3 (70 g, 132.59 mmol). The reaction mixture was then heated to 70 °C and reacted for 6 hours. The reaction solution was cooled to 45 °C, followed by the addition of water (240 mL) for dilution. Ethanol was removed by rotary evaporation under reduced pressure. Methyl *tert*-butyl ether (400 mL) and heptane (200 mL) were added to a beaker, and the two solvents were mixed thoroughly for subsequent use. Approximately half of the above-mentioned mixed solvent was added to the residue from reduced-pressure rotary evaporation. The mixture was stirred for 5 minutes, allowed to stand, and the upper organic phase was separated, and the aqueous phase was retained. The remaining half of the above-mentioned mixed solvent was added to the aqueous phase. The mixture was stirred for 5 minutes, allowed to stand, and then the upper organic phase was separated and the aqueous phase was retained. Dilute hydrochloric acid (2 M, purchased from Chengdu Kelong) was added to the retained aqueous phase to adjust the pH≤3. Ethyl acetate was used for extraction (500 mL). The organic phase was washed with water (200 mL) and saturated brine (200 mL), respectively. The organic phase was concentrated under reduced pressure to obtain the target product as a brownish-yellow oil (58.03 g, 99.96%). MS (ESI, pos. ion) m/z: 438.1 (M+1).

A scale-up experiment was carried out according to the method described in this example. With a feed amount of 3.525 kg of intermediate 3, the target product could still be obtained with high yield and high purity, which was 2.757 kg of a brownish-yellow oil with a yield of 100%.

### Example 5 Preparation of (S)-methyl 1-(2-(benzyloxy)ethyl)-2-chloro-4-methyl-5-(2-(trifluoromethyl)phenyl)-1H-pyrrole-3-carboxamide (intermediate 5)

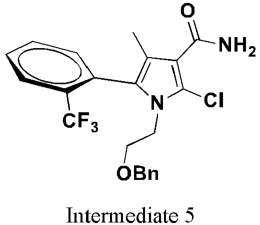

The intermediate 4 (66 g, 150.74 mmol), *N*,*N*-dimethylacetamide (135 mL) and 1-(1H-imidazolium-1-carbonyl)-1*H*-imidazolium (36.66 g, 226.11 mmol) were added to the reaction flask. The mixture was reacted at room temperature for 2 hours, and then to which was added ammonia (312.91 g, 2410.4 mmol). after the addition was completed, the mixture was stirred at room temperature for 20 hours. The reaction solution was diluted with water (300 mL) and extracted with isopropyl acetate (200 mL × 2). The organic phase was washed with water (300 mL) and saturated brine (300 mL) and concentrated under reduced pressure to obtain the target product as a brownish-yellow oil (65.80 g, 99.99%). MS (ESI, pos. ion) m/z: 437.5 (M+1).

A scale-up experiment was carried out according to the method described in this example. With a feed amount of 2.740 kg of intermediate 4, the target product could still be obtained with high yield and high purity, which was 2.734 kg of a brownish-yellow oil with a yield of 100%, a purity of 99.50%, and 1.90% of an isomer.

### Example 6 Preparation of (S)-1-(2-(benzyloxy)ethyl)-4-methyl-5-(2-(trifluoromethyl)phenyl)-1H-pyrrole-3-carboxamide (intermediate 6)

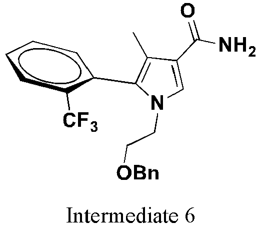

### Instance 1:

The intermediate 5 (18.0 g, 41.20 mmol), EtOH (108 mL), toluene (54 mL), water (9 mL), sodium formate (3.36 g, 49.44 mmol) and palladium-carbon (0.543 g, 10%) were added to a reaction flask. The mixture was then heated to 60 °C and reacted for 4.5 hours. The reaction solution was filtered through a celite pad, and the filter cake was washed with ethanol (50 mL). *N*,*N*-dimethylacetamide (70 mL) was added to the filtrate, and the mixture was concentrated under reduced pressure to remove ethanol and toluene. Water (100 mL) was added dropwise to the distillation residue, and the mixture was stirred at room temperature for 2 hours. The mixture was filtered under suction, and the filter cake was washed with water (60 mL), collected, and vacuum dried at 60 °C for 18 hours to obtain the target product as an off-white solid (15.60 g, 94.08%) with a purity of 99.01% and 1.81% of an isomer.

MS (ESI, pos. ion) m/z: 403.3 (M+1).

¹H NMR (400 MHz, DMSO-*d₆*) δ (ppm) 7.91 - 7.80 (m, 1H), 7.71 - 7.63 (m, 2H), 7.50 (s, 1H), 7.35 - 7.25 (m, 4H), 7.20 (d, *J=* 6.9 Hz, 2H), 7.09 (s, 1H), 6.62 (s, 1H), 4.44 - 4.34 (m, 2H), 3.82 - 3.73 (m, 1H), 3.58 - 3.41 (m, 3H), 1.86 (s, 3H).

¹⁹F NMR (376 MHz, DMSO-*d₆*) δ (ppm) -59.58 (s).

Instance 2-11

Following a method similar to Instance 1, the reaction conditions for preparing intermediate 6 were tested, wherein the reaction temperature was 60 °C and the amount of sodium formate was 1.2 equivalents (i.e., the molar amount of sodium formate was 1.2 times the molar amount of intermediate 5). The experimental results are shown in Table A below.

**Table A**

| Instance | Feed Amount | Palladium-carbon | Solvent | Reaction Time | In-process Product Content | Yield |
|---|---|---|---|---|---|---|
| 2 | 1.38 g | 5% | ethanol | 2h | 93.66% | N/A |
| 3 | 1.41 g | 3% | ethanol | 3 h | 92.05% | N/A |
| 4 | 1.43 g | 5% | Toluene/water = 10/1 | 2h | No reaction | N/A |
| 5 | 3.36 g | 3% | Ethanol/toluene/water = 8/2/1 | 3 h | 94.84% | N/A |
| 6 | 1.45 g | 3% | Ethanol/toluene/water = 5/5/1 | 3 h | 89.91% | N/A |
| 7 | 10.7 g | 3% | Ethanol/toluene/water = 12/6/1 | 4 h | 93.65% | N/A |
| 8 | 15.2 g | 3% | Ethanol/toluene/water = 16/4/1 | 3 h | 94.70% | N/A |
| 9 | 4.82 g | 3% | Ethanol/toluene/water = 12/6/1 | 3 h | 94.30% | 81.18% |
| 10 | 11.0 g | 5% | Ethanol/toluene/water = 10/10/1 | 3 h | 90.50% | 82.50% |
| 11 | 32 g | 3% | Ethanol/toluene/water = 12/6/1 | 3 h | 94.97% | 84.76% |

| | | | | | | |
|---|---|---|---|---|---|---|
| Notes: In the table above, the feed amount refers to the amount of intermediate 5; the amount of palladium-carbon is calculated as a mass percentage of raw material intermediate 5; the ratio of mixed solvent refers to the volume ratio; the in-process product content refers to the product content in the reaction system after the reaction but before post-treatment, which is the peak area ratio of the product obtained by HPLC detection of samples taken from the reaction system; the yield refers to the yield of the target product obtained after the reaction and post-treatment. The post-treatment method adopts the DMAC/H₂O system and refers to the method in Instance 1. The post-treatment method of the DMAC/H₂O system has a good impurity removal effect, and the product purity is high (up to 98% or above), which can save the additional recrystallization operation, thereby saving time and cost; N/A means no corresponding data. | | | | | | |

A scale-up experiment was carried out according to the method described in this example. With a feed amount of 2.615 kg of intermediate 5, the target product could still be obtained with high yield and high purity, which was 2.152 kg of an off-white solid, with a yield of 89.4%, a purity of 99.42%, and 1.25% of an isomer.

The compound of formula (A) can be prepared by using intermediate 6 as a raw material according to the methods described in other parts of this invention (including but not limited to the methods described in Examples 7-8 below) or similar methods disclosed in the prior arts.

### Example 7 Preparation of (S)-1-(2-(benzyloxy)ethyl)-N-(3-fluoro-4-methylsulfonyl)phenyl)-4-methyl-5-(2-(trifluoromethyl)phenyl)-1H -pyrrole-3-carboxamide (intermediate 7)

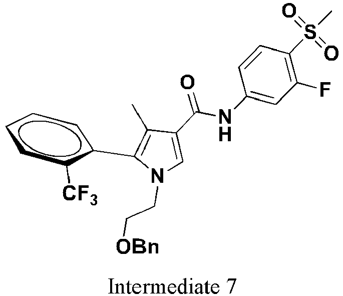

The intermediate 6 (15.40 g, 38.27 mmol), 4-bromo-2-fluoro-1-methanesulfonylbenzene (9.98 g, 39.42 mmol), palladium acetate (0.21 g, 0.96 mmol), 4,5-bis(diphenylphosphine-9,9-dimethyloxanthracene) (0.55 g, 0.96 mmol), cesium carbonate (24.94 g, 76.54 mmol) and 2-methyl-2-butanol (*tert*-amyl alcohol) (125 mL) were added to the reaction flask. After the additions, the mixture was replaced with nitrogen four times, and heated to 65 °C for 3 hours under a nitrogen atmosphere. Water (80 mL) was added to the reaction flask, and the mixture was stirred for 3 minutes. Isopropyl acetate (50 mL) was then added, and the mixture was stirred for 5 minutes. After standing for phase separation, the upper organic phase was retained. The organic phase was filtered through diatomaceous earth under suction, and the filter cake was washed with isopropyl acetate (35 mL). The filtrate was washed with 20% saline solution (300 mL × 2) and concentrated under reduced pressure to obtain the crude product. Ethanol (88 mL), palladium-free silica gel (4.25 g) and activated carbon (1.64 g) were added to the crude product, and the mixture was heated to 70 °C and stirred for 1 hour. Heating was turned off, and the mixture was allowed to cool to room temperature before stirring for 10 hours. The mixture was filtered through a celite pad under suction, and the filter cake was washed with ethanol (44 mL). The filtrate was retained for further use. The filtrate was added dropwise to water (680 mL) under ice bath conditions. After the addition was complete, the mixture was allowed to warm to room temperature and stirred for 3 hours. The mixture was filtered under suction, and filter cake was washed with water (40 mL), collected, and vacuum dried at 45 °C for 40 hours to obtain the target product as an off-white solid (19.21 g, 87.36%) with a purity of 99.01% and 94.04% of an isomer.

### Example 8 Preparation of (S)-1-(2-hydroxyethyl)-N-(3-fluoro-4-methylsulfonyl)phenyl)-4-methyl-5-(2-(trifluoromethyl)phenyl)-1H-py rrole-3-carboxamide (the compound of formula (A))

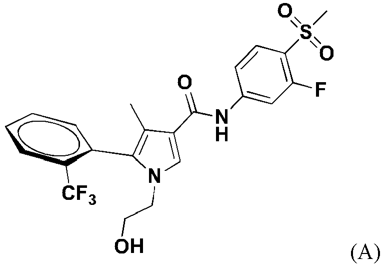

The intermediate 7 (24.6 g, 42.81 mmol), ethanol (245 mL), palladium-carbon (2.47 g, 10%) and 6 M HCl (4.0 mL) were added to the reaction flask. After the addition was completed, the mixture was heated to 55±5 °C for 6 hours under a hydrogen atmosphere. Heating was turned off, and the mixture was allowed to cool to room temperature. The reaction solution was filtered through a celite pad. The filter cake was washed with ethanol (45 mL), and the filtrate was concentrated under reduced pressure. Ethanol (90 mL) was added to the residue, and the mixture was added dropwise to water (730 mL) after dissolving. After the addition was complete, the mixture was stirred at room temperature for 1 hour. The mixture was filtered under suction, and filter cake was washed with water (50 mL), collected, and vacuum dried at 50 °C for 46 hours to obtain the crude product. Isopropyl acetate (62 mL) and toluene (62 mL) were added to the crude product. The mixture was heated to 55±5 °C, and then to which was added toluene (188 mL) dropwise. After the addition was complete, the mixture was kept warm and stirred until a solid precipitated, then stirred for another 3 hours. Toluene (62 mL) was added dropwise, and the mixture was stirred for 2 hours. Heating was turned off, and the mixture was allowed to cool to room temperature before stirring for 13 hours. The mixture was filtered under suction, and the filter cake was washed with toluene (41 mL), collected, and vacuum dried at 60 °C for 23 hours to obtain the target product as an off-white solid (14.99 g, 72.27%) with a purity of 99.39% and 0.33% of an isomer.

MS (ESI, pos. ion) m/z: 485.2 (M+1).

¹H NMR (400 MHz, DMSO-*d₆*) δ (ppm) 10.15 (s, 1H), 7.98 (d*, J =* 13.5 Hz, 1H), 7.89 (d, *J* = 7.8 Hz, 1H), 7.81 (s, 1H), 7.78 (d, *J=* 8.2 Hz, 2H), 7.71 (t*, J =* 8.3 Hz, 2H), 7.47 (d, *J =* 7.4 Hz, 1H), 4.95 (t, *J* = 4.8 Hz, 1H), 3.69 (dt*, J* = 10.6, 6.8 Hz, 1H), 3.51 (pd, *J* = 10.6, 5.4 Hz, 3H), 3.28 (s, 3H), 1.93 (s, 3H).

¹⁹F NMR (376 MHz, DMSO-*d₆*) δ (ppm) -59.56 (s), -109.31 (s).

Reference throughout this specification to "an embodiment," "some embodiments," "one embodiment", "another example," "an example," "a specific examples," or "some examples," means that a particular feature, structure, material, or characteristic described in connection with the embodiment or example is included in at least one embodiment or example of the present disclosure. Thus, the appearances of the phrases such as "in some embodiments," "in one embodiment", "in an embodiment", "in another example, "in an example," "in a specific examples," or "in some examples," in various places throughout this specification are not necessarily referring to the same embodiment or example of the present disclosure.

Although explanatory embodiments have been shown and described, it would be appreciated by those skilled in the art that the above embodiments cannot be construed to limit the present disclosure, and changes, alternatives, and modifications can be made in the embodiments without departing from spirit, principles and scope of the present disclosure.

## Claims

1. A compound of Formula (I), 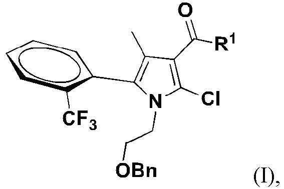 wherein, R¹ is OH, NH₂, C₂₋₄ alkoxy or benzyloxy, each of the C₂₋₄ alkoxy and benzyloxy is optionally substituted with 1, 2, 3 or 4 substituents independently selected from halogen, C₁₋₄ alkyl, C₁₋₄ haloalkyl, C₁₋₄ alkoxy and C₁₋₄ haloalkoxy.

2. A compound having one of the following structures,

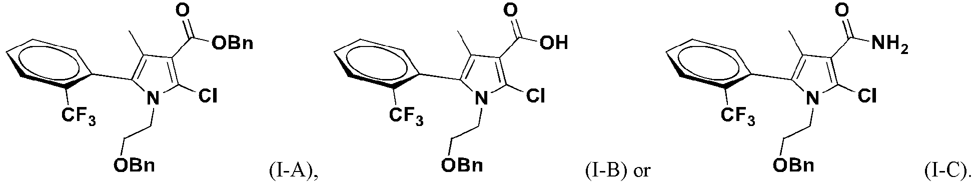

3. A method for preparing the compound of Formula (II), 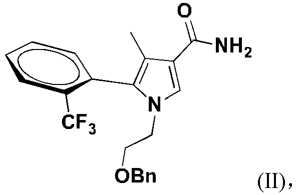 wherein, the method comprises:
Step d) The compound of formula (I-C) is dechlorinated in a solvent under suitable conditions to obtain the compound of formula (II).

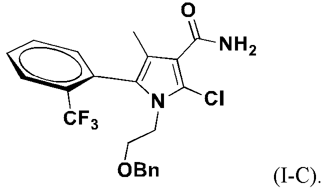

4. The method according to claim 3, wherein, the solvent is methanol, ethanol, isopropanol, tert-butanol, or a mixed solvent composed of any of them with other solvent in any combination, and the other solvent is DMF, tetrahydrofuran, ethyl acetate, methyl *tert*-butyl ether, ethylene glycol dimethyl ether, ethylene glycol monomethyl ether, toluene, water, or any combination thereof;
optionally, the solvent is methanol, ethanol, ethanol/toluene, ethanol/water, ethanol/toluene/water, ethanol/tetrahydrofuran, or ethanol/tetrahydrofuran/water.

5. The method according to claim 3 or 4, wherein, the reaction in step d) is carried out in the presence of a transition metal catalyst, optionally, the transition metal catalyst is a palladium-carbon catalyst, a palladium acetate catalyst or a nickel catalyst.

6. The method according to any one of claims 3-5, wherein, the reaction in step d) is carried out in the presence of ammonium formate, sodium formate, disodium hydrogen phosphate, acid, triethylamine or hydrogen.

7. The method according to any one of claims 3-6, wherein, the reaction in step d) is carried out under heating conditions; optionally, the heating conditions are heating to 45 °C-75 °C, 55 °C-70 °C or 60 °C-65 °C.

8. The method according to any one of claims 3-7, further comprising a method for preparing the compound of formula (I-C), wherein, the method for preparing the compound of formula (I-C) comprises:
Step a) The compound of formula (I-1) reacts with a benzyl protecting group reagent to obtain the compound of formula (I-A); 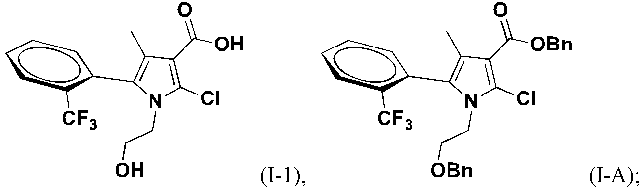
Step b) The compound of formula (I-A) is debenzylated under suitable conditions to obtain the compound of formula (I-B); 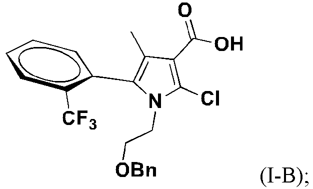
Step c) The compound of formula (I-B) is aminated under suitable conditions to obtain the compound of formula (I-C).

9. The method according to claim 8, wherein, the benzyl protecting group reagent in step a) is a halobenzyl group, optionally benzyl bromide;
optionally, the step a) is carried out in the presence of sodium *tert*-amylate;
optionally, the reaction solvent in step a) is *N*,*N*-dimethylacetamide;
optionally, the reaction temperature in step a) is 25 °C to 60 °C; preferably, the reaction temperature in step a) is 35 °C-55 °C, 35 °C-45 °C or 45 °C-55 °C.

10. The method according to claim 8 or 9, wherein, the reaction in step b) is carried out in the presence of a base, wherein the base is sodium carbonate, potassium carbonate, cesium carbonate, potassium phosphate, lithium hydroxide, potassium hydroxide or sodium hydroxide,
optionally, the reaction in step b) is carried out in a solvent, and the solvent is acetone, acetonitrile, *N*,*N*-dimethylformamide, *N*,*N*-dimethylacetamide, DMSO, methanol, ethanol, THF, methyl *tert*-butyl ether, water or any combination thereof;
optionally, the reaction in step b) is carried out under heating conditions, and the heating conditions refer to heating to 60 °C to 80 °C, or, heating to 65 °C to 75 °C.

11. The method according to any one of claims 8-10, wherein, in step c), the compound of formula (I-B) reacts with thionyl chloride, oxalyl chloride or 1-(1*H*-imidazolium-1-carbonyl)-1*H*-imidazolium and then reacts with an amination reagent to obtain the compound of formula (I-C); optionally, the amination reagent is ammonia or an ammonium salt reagent, preferably, the amination reagent is ammonia, ammonium bromide or NH₄SCN.

12. The method according to any one of claims 8-11, wherein, the reaction in step c) is carried out in a solvent, and the solvent is *N*,*N*-dimethylformamide or *N*,*N*-dimethylacetamide; optionally, the reaction in step c) is carried out at room temperature.

13. The method according to any one of claims 8-12, further comprising a method for preparing the compound of formula (I-1), wherein, the method for preparing the compound of formula (I-1) comprises:
1a) The compound of formula (I-0) reacts with an optically active amine to obtain the corresponding salt of the photoactive amine of the compound of formula (I-1), and
1b) The salt obtained in step 1a) reacts to give the compound of formula (I-1);

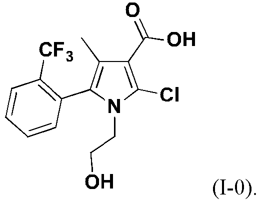

14. The method according to claim 13, wherein, the optically active amine is an optically active amine having a quinine skeleton; optionally, the optically active amine having a quinine skeleton is quinine, hydroquinine, quinidine, cinchonine or cinchonidine.

15. The method according to claim 13 or 14, wherein, the reaction in step 1b) is a hydrolysis reaction;
optionally, the reaction in step 1b) is carried out under acidic conditions; optionally, the acidic conditions are the conditions in the presence of hydrochloric acid, sulfuric acid, hydrobromic acid or citric acid;
optionally, the reaction solvent in step 1b) is *N*,*N*-dimethylformamide, *N*,*N*-dimethylacetamide, acetonitrile, tetrahydrofuran, ethanol, acetone, isopropyl acetate, ethyl acetate, methyl *tert*-butyl ether, water, dimethoxyethane or any combination thereof.

16. The method according to any one of claims 13-14, wherein, the reaction solvent in step 1a) is *N*,*N*-dimethylformamide, *N*,*N*-dimethylacetamide, acetonitrile, tetrahydrofuran, ethanol, acetone, isopropyl acetate, ethyl acetate, methyl tert-butyl ether, water, dimethoxyethane or any combination thereof;
optionally, the reaction temperature in step 1a) is from room temperature to 100 °C; preferably, the reaction temperature in step 1a) is 50 °C-80 °C or 55 °C-65 °C.

17. Use of a compound having one of the following structures in the preparation of a pyrrole amide compound used as a mineralocorticoid receptor antagonist,

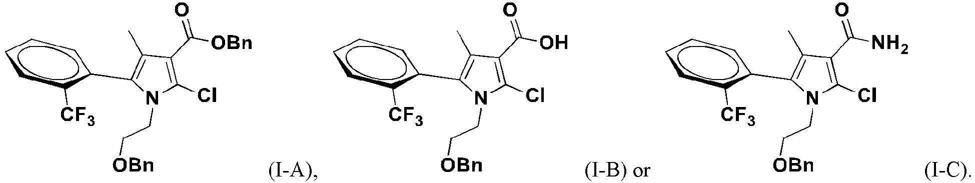

18. The use according to claim 17, wherein, the pyrrole amide compound used as a mineralocorticoid receptor antagonist is (*S*)-*N*-(3-fluoro-4-(methylsulfonyl)phenyl)-1-(2-hydroxyethyl)-4-methyl-5-(2-(trifluoromethyl)phenyl)-1*H*-pyrrol e-3-carboxamide.

19. A method for preparing (*S*)-*N*-(3-fluoro-4-(methylsulfonyl)phenyl)-1-(2-hydroxyethyl)-4-methyl-5-(2-(trifluoromethyl)phenyl)-1*H*-pyrrol e-3-carboxamide, comprising: the compound of formula (II) reacts with the compound of formula (III) to obtain the compound of formula (IV), and the compound of formula (IV) undergoes a deprotection reaction to remove the benzyl group to obtain the target compound; 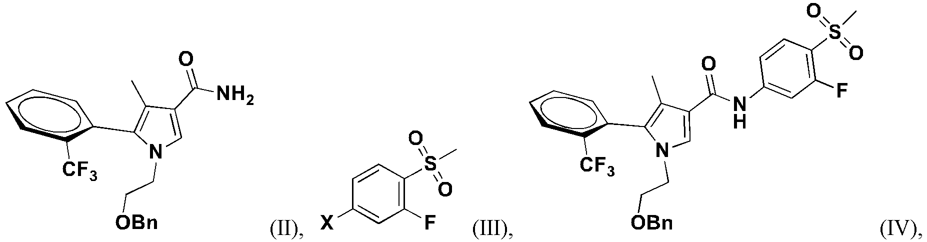 wherein, X is Br or I;
wherein, the method further comprises a method for preparing the compound of Formula (II) according to any one of claims 3-16.

20. The method according to claim 19, wherein, the deprotection reaction is carried out in a hydrogen atmosphere in the presence of palladium-carbon; optionally, the deprotection reaction is carried out under acidic conditions or in the presence of an acidic substance, wherein the acidic conditions refer to conditions in the presence of an acid, preferably, the acid is hydrochloric acid, sulfuric acid, trifluoroacetic acid or a solution thereof;
optionally, the reaction temperature of the deprotection reaction is 50 °C-75 °C or 55 °C-70 °C;
optionally, the deprotection reaction is carried out in an alcohol solvent, and the alcohol solvent is methanol, ethanol, isopropanol, *tert*-butanol or *tert*-amyl alcohol.

21. The reaction according to claim 20, wherein, the amount of acid used is from 0.10 mL/g to 0.40 mL/g, or from 0.10 mL/g to 0.20 mL/g relative to the mass of the compound of formula (IV).

22. The reaction according to any one of claims 19-21, wherein, the reaction of the compound of formula (II) with the compound of formula (III) is carried out in the presence of a base, a catalyst and a ligand, optionally, the base is potassium carbonate, cesium carbonate, sodium carbonate, potassium *tert*-butoxide or potassium phosphate, the catalyst is Pd₂(dba)₃, Pd(dppf)Cl₂·CH₂Cl₂ or Pd(OAc)₂; and the ligand is Xantphos, X-phos or S-phos;
optionally, the reaction of the compound of formula (II) with the compound of formula (III) is carried out in a solvent, wherein the solvent is toluene, dioxane, *tert*-butanol, *tert*-amyl alcohol, dimethyl ether, cyclopentyl methyl ether, *N*,*N*-dimethylacetamide, *N*,*N*-dimethylformamide, water or any combination thereof.
optionally, the temperature of the reaction of the compound of formula (II) with the compound of formula (III) is 55 °C-110 °C, preferably 60 °C-70 °C.
